# EUROPEAN PATENT APPLICATION

(11) **EP 4 279 924 A1**
(43) Date of publication of application: **22.11.2023**
(21) Application number: 22739526.6
(22) Date of filing: 17.01.2022
(51) Int. Cl.: G01N 33/68

(54) **ADSORPTION INHIBITOR FOR HEPCIDIN, METHOD FOR INHIBITING ADSORPTION, REFERENCE STANDARD, REAGENT, KIT AND MEASUREMENT METHOD**

(30) Priority: 18.01.2021 JP 2021005702
(71) Applicant: FUJIFILM Corporation, Tokyo 106-8620 (JP)
(72) Inventor: TAKAGAKI Takashi, Amagasaki-shi, Hyogo 661-0963 (JP); ITAI Tomokazu, Amagasaki-shi, Hyogo 661-0963 (JP)
(74) Representative: Hughes, Andrea Michelle
(86) International application number: PCT/JP2022/001421
(87) International publication number: WO 2022/154121

(57) **Abstract**

An object of the present invention is to provide a hepcidin adsorption inhibitor, a method of inhibiting adsorption, a standard product, a reagent, a kit, and a measuring method. The present invention relates to a hepcidin adsorption inhibitor including a chelating agent, a method of inhibiting adsorption of hepcidin, which includes bringing a hepcidin-containing liquid into contact with at least one member selected from a rubber member, a plastic member, or a glass member, in a presence of a chelating agent, a standard product for measuring hepcidin, which contains the hepcidin adsorption inhibitor and hepcidin, and the like. According to the present invention, the adsorption of hepcidin is inhibited.

## Description

### BACKGROUND OF THE INVENTION

### 1. Field of the Invention

The present invention relates to a hepcidin adsorption inhibitor, a method of inhibiting adsorption, a standard product, a reagent, a kit, and a measuring method.

### 2. Description of the Related Art

Hepcidin is a peptide hormone that is mainly produced and secreted by hepatocytes and plays a central role in maintaining iron homeostasis (J Biol Chem 2001; 276: 7806-10). Hepcidin is encoded as a prepropeptide of 84 amino acids. It is processed into prohepcidin of 60 amino acids, and then this prohepcidin undergoes proteolysis to form hepcidin 25 of 25 amino acids having a physiological activity (J Biol Chem 2002; 277: 37597-603). In the amino acid sequence of hepcidin 25, the proportion of cysteine residues which are hydrophobic amino acids is high, and four disulfide bonds are formed by eight cysteine residues.

As a peptide adsorption inhibitor, a serum protein or the like, represented by bovine serum albumin (BSA), is commonly used.

### SUMMARY OF THE INVENTION

As a result of studying a reagent used for measuring hepcidin, the inventors of the present invention found that there is a problem that an accurate measured value of a hepcidin concentration cannot be obtained since hepcidin is adsorbed to various containers. In addition, the inventors of the present invention confirmed that a substance known as the peptide adsorption inhibitor, such as BSA, is insufficient in the effect of inhibiting the adsorption of hepcidin to a container.

In consideration of the above circumstances, an object of the present invention is to provide a hepcidin adsorption inhibitor, a method of inhibiting adsorption, a standard product, a reagent, a kit, and a measuring method.
[1] A hepcidin adsorption inhibitor, comprising a chelating agent.
[2] The hepcidin adsorption inhibitor according to [1], in which the chelating agent is an aminocarboxylic acid-based chelating agent or a phosphonic acid-based chelating agent.
[3] The hepcidin adsorption inhibitor according to [1] or [2], in which adsorption inhibition of the hepcidin adsorption inhibitor is inhibition of adsorption to a rubber member.
[4] The hepcidin adsorption inhibitor according to [3], in which the rubber member is a synthetic rubber member.
[5] The hepcidin adsorption inhibitor according to [4], in which the synthetic rubber member is a butyl rubber member.
[6] The hepcidin adsorption inhibitor according to [1] or [2], in which adsorption inhibition of the hepcidin adsorption inhibitor is inhibition of adsorption to a plastic member or a glass member.
[7] A method of inhibiting adsorption of hepcidin, comprising bringing a hepcidin-containing liquid into contact with at least one member selected from a rubber member, a plastic member, or a glass member, in a presence of a chelating agent.
[8] The method of inhibiting adsorption of hepcidin according to [7], in which the chelating agent is an aminocarboxylic acid-based chelating agent or a phosphonic acid-based chelating agent.
[9] The method of inhibiting adsorption of hepcidin according to [7] or [8], in which the member is a rubber member.
[10] The method of inhibiting adsorption of hepcidin according to [9], in which the rubber member is a synthetic rubber member.
[11] The method of inhibiting adsorption of hepcidin according to [10], in which the synthetic rubber member is a butyl rubber member.
[12] The method of inhibiting adsorption of hepcidin according to [7] or [8], in which the member is a plastic member or a glass member.
[13] A standard product for measuring hepcidin, comprising:
   the hepcidin adsorption inhibitor according to any one of [1] to [6]; and
   hepcidin.
[14] A reagent for measuring hepcidin, comprising the hepcidin adsorption inhibitor according to any one of [1] to [6].
[15] A reagent kit for measuring hepcidin, in which the hepcidin adsorption inhibitor according to any one of [1] to [6] includes a hepcidin adsorption inhibitor that is accommodated in a measuring instrument having at least one member selected from a rubber member, a plastic member, or a glass member.
[16] A measuring method for hepcidin, in which the standard product for measuring hepcidin according to [13] and/or the reagent for measuring hepcidin according to [14] is used.

According to the present invention, it is possible to inhibit the adsorption of hepcidin. Specifically, it is possible to inhibit the adsorption of hepcidin in a hepcidin-containing liquid.

### DESCRIPTION OF THE PREFERRED EMBODIMENTS

In the present specification, in a case where an upper limit and a lower limit of a range are indicated as A to B, it indicates that it is equal to or larger than A or equal to or less than B, unless otherwise specified. In addition, the measurement in the present specification includes quantitative measurement, semi-quantitative measurement, and qualitative measurement.

### <Hepcidin adsorption inhibitor according to embodiment of present invention>

The hepcidin adsorption inhibitor according to the embodiment of the present invention contains a chelating agent as an active ingredient. According to the hepcidin adsorption inhibitor according to the embodiment of the present invention, it is possible to inhibit the adsorption of hepcidin to at least one member selected from a rubber member, a plastic member, or a glass member. Specifically, it is possible to inhibit the adsorption of hepcidin in a hepcidin-containing liquid.

The hepcidin according to the embodiment of the present invention may be any protein that is obtained from a hepcidin prepropeptide consisting of 84 amino acids and a hepcidin prepropeptide through processing or proteolysis, and examples thereof include hepcidin 25, hepcidin 22, and hepcidin 20. The hepcidin according to the embodiment of the present invention may be obtained from a biological specimen or may be produced according to a gene recombination method.

Examples of the chelating agent according to the embodiment of the present invention include an aminocarboxylic acid-based chelating agent (a chelating agent having a nitrogen atom and a carboxy group) and a phosphonic acid-based chelating agent, where examples of the aminocarboxylic acid-based chelating agent include Bicine as a chelating agent having one carboxy group; as a chelating agent having two carboxy groups, ethylenediaminediacetic acid (EDDA), ethylenediaminedipropionic acid (EDDP), iminodiacetic acid (IDA), or hydroxyethyliminodiacetic acid (HIDA); as a chelating agent having three carboxy groups, nitrilotriacetic acid (NTA), hydroxyethylethylenediamine triacetate (EDTA-OH; (HEDTA)), or nitrilotripropionic acid (NTP); as a chelating agent having four carboxy groups, ethylenediamine tetraacetic acid (EDTA), trans-1,2-diaminocyclohexane-N,N,N',N'-tetraacetic acid (CyDTA), diaminopropanol tetraacetic acid (DPTA-OH), glycol ether diamine tetraacetic acid (EGTA) (GEDTA), 1,2-bis(o-aminophenoxy)ethane-N,N,N',N'-tetraacetic acid (BAPTA), or diaminopropanetetraacetic acid (Methyl-EDTA); as a chelating agent having five carboxy groups, diethylenetriamine-N,N,N',N",N"-pentaacetic acid (DTPA); as a chelating agent having six carboxy groups, triethylenetetraminehexacetic acid (TTHA); and salts of the above-described compounds (for example, an alkali metal salt, an alkaline earth metal salt, and an ammonium salts), and examples of the phosphonic acid-based chelating agent include a phosphonic acid-based chelating agent having a nitrogen atom, such as ethylenediaminetetraethylenephosphonic acid (EDTPO) or nitrilotris(methylene phosphonic acid) (NTPO); and a salt of the above-described compound (for example, an alkali metal salt, an alkaline earth metal salt, or ammonium salt), among which the preferred ones are a phosphonic acid-based chelating agent having a nitrogen atom or an aminopolycarboxylic acid-based chelating agent (an aminopolycarboxylic acid-based chelating agent having two or more carboxy groups), and a salt of thereof (for example, an alkali metal salt, an alkaline earth metal salt, or an ammonium salts). The aminopolycarboxylic acid-based chelating agent is more preferably an aminopolycarboxylic acid-based chelating agent having 2 or more and 6 or less carboxy groups. In addition, in a case where the member according to the embodiment of the present invention is a rubber member or a glass member, a phosphonic acid-based chelating agent having a nitrogen atom, an aminopolycarboxylic acid-based chelating agent having 2 or more and 4 or less carboxy groups, and salts thereof are still more preferable, and EDTA, EDTPO, NTPO, EDDA, NTA, and BAPTA are particularly preferable. In a case where the member according to the embodiment of the present invention is a plastic member, a phosphonic acid-based chelating agent having a nitrogen atom, an aminopolycarboxylic acid-based chelating agent having 3 or more and 6 or less carboxy groups, and salts thereof are still more preferable, and EDTA, EDTPO, NTPO, NTA, BAPTA, CyDTA, GEDTA, DTPA, and TTHA are particularly preferable. One kind of the chelating agent according to the embodiment of the present invention may be used alone, or two or more kinds thereof may be used in combination.

The hepcidin adsorption inhibitor according to the embodiment of the present invention may contain other components other than the chelating agent according to the embodiment of the present invention, as necessary. Examples of other components include a preservative (for example, sodium azide, salicylic acid, or benzoic acid) and a reaction accelerator. The hepcidin adsorption inhibitor according to the embodiment of the present invention may be in a solid state or may be in a liquid state, for example, in water such as deionized water or distilled water, a buffer solution, a biological specimen, or the like, or a solution or suspension contained in a liquid or the like, which contains water or a buffer solution, and a biological specimen. The buffer solution and the biological specimen are the same as those described above, and the same applies to specific examples and preferred ones thereof. In addition, the hepcidin adsorption inhibitor according to the embodiment of the present invention may be used in combination with a peptide adsorption inhibitor such as a surfactant or albumin (for example, bovine serum albumin).

The member according to the embodiment of the present invention constitutes a part or all of an instrument (hereinafter, a measuring instrument according to the present invention) that is used for measuring hepcidin, and a surface that comes into contact with hepcidin is composed of a material to which hepcidin is adsorbed, where the material is at least one selected from rubber, plastic, or glass. The measuring instrument according to the present invention is an instrument with which a liquid containing hepcidin (the hepcidin-containing liquid according to the present invention) comes into contact in the measurement of hepcidin. In the measuring instrument according to the present invention, a surface or a part of the surface with which hepcidin comes into contact is composed of the member according to the embodiment of the present invention. The member according to the embodiment of the present invention which "is composed of a material to which hepcidin is adsorbed (is made of a material to which hepcidin is adsorbed)" may be a member in which only a part of a surface that comes into contact with hepcidin is composed of a material to which hepcidin is adsorbed or may be a member in which the entire surface that comes into contact with hepcidin is composed of a material to which hepcidin is adsorbed. In addition, the measuring instrument according to the present invention includes not only a measuring instrument in which only the surface that comes into contact with hepcidin is composed of a material to which hepcidin is adsorbed but also a measuring instrument in which the entire member according to the embodiment of the present invention is composed of a material to which hepcidin is adsorbed.

Examples of the rubber include synthetic rubber such as butyl rubber, silicone rubber, fluorinated silicone rubber, fluororubber, isoprene rubber, butadiene rubber, chloroprene rubber, nitrile rubber, butyl rubber, acrylic rubber, and urethane rubber, and natural rubber, where synthetic rubber is preferable, and butyl rubber is more preferable. Examples of the plastic include polyethylene (PE), polypropylene (PP), polystyrene (PS), polyvinyl chloride (PVC), polyurethane (PU), polytetrafluoroethylene (PTFE), polyethylene terephthalate (PET), an acrylic resin such as polymethyl methacrylate (PMMA), and a copolymer of polyethylene and polypropylene, where polyethylene (PE), polypropylene (PP), or polystyrene (PS) is preferable.

The form of the member according to the embodiment of the present invention or/and the measuring instrument according to the present invention are not particularly limited, and examples thereof include a container (hereinafter, described as a container according to the present invention) such as a vial bottle, a tube (a microtube), a test tube, a microtiter plate (an ELISA plate), a microarray substrate, a tray, or a cell, a container lid, such as a stopper or a cap, a bead, a particle, a pipette, a tip, and a disk-shaped piece. The container according to the present invention is a container that is capable of accommodating hepcidin, and it is used for measuring hepcidin. The container according to the present invention is preferably a container having a surface composed of plastic (a plastic container) or a container having a surface composed of glass (a glass container), more preferably a polyethylene (PE) container, a polypropylene (PP) container, a polystyrene (PS) container, or a glass container, and still more preferably a polypropylene (PP) container, a polyethylene (PE) container, or a glass container. The container according to the present invention may have a container lid which is one form of the member according to the embodiment of the present invention, and it is preferably a container having a container lid. The shape of the container lid according to the present invention is not particularly limited as long as it closes the opening of the container according to the present invention. The container lid according to the present invention is preferably a lid having a surface composed of rubber (a rubber lid) or a lid having a surface composed of plastic (a plastic lid), more preferably a rubber lid, still more preferably synthetic rubber lid, and particularly preferably a butyl rubber lid.

The hepcidin-containing liquid according to the present invention is a liquid containing hepcidin, and it may be a solution or may be a suspension as long as it contains hepcidin. Examples of the hepcidin-containing liquid according to the present invention include a solution or suspension obtained by incorporating hepcidin in water such as deionized water or distilled water or in a buffer solution, a biological specimen containing hepcidin, and a solution or suspension obtained by incorporating hepcidin in water or in a liquid containing a buffer solution and a biological specimen. Specifically, examples of the hepcidin-containing liquid according to the present invention include a standard product such as a calibrator or a control, which is used for the creation of a calibration curve in various measuring methods such as an immunological measuring method, a mass spectrometry method, and the like, the checking of the effectiveness of the accuracy control and calibration of analytical instruments, and the investigation of the stability of the temporal quantitative analysis, a reagent for measuring hepcidin, and a biological specimen obtained from a test animal or the like. Examples of the test animal according to the present invention include mammals such as a human, a monkey, a mouse, a rat, a dog, a cat, a pig, a rabbit, and a chimpanzee, where a human, a monkey, a mouse, or a rat is preferable, and a human is more preferable.

Examples of the buffer solution include (Good's) buffer solutions obtained by dissolving buffering agents such as N-(2-acetoamido)-2-aminoethanesulfonic acid (ACES), N-(2-acetoamido)iminodiacetic acid (ADA), N,N-bis(2-hydroxyethyl)-2-aminoethanesulfonic acid (BES), N,N-bis(2-hydroxyethyl)glycine (Bicine), bis(2-hydroxyethyl)iminotris(hydroxyethyl)methane (Bis-Tris), N-cyclohexyl-3-aminopropanesulfonic acid (CAPS), N-cyclohexyl-2-hydroxy-3-aminopropanesulfonic acid (CAPSO), N-cyclohexyl-2-aminoethanesulfonic acid (CHES), 3-[N,N-bis(2-hydroxyethyl)amino]-2-hydroxypropanesulfonic acid (DIPSO), 3-[4-(2-hydroxyethyl)-1-piperazinyl]propanesulfonic acid (EPPS), 2-[4-(2-hydroxyethyl)-1-piperazinyl]ethanesulfonic acid (HEPES), 2-hydroxy-3-[4-(2-hydroxyethyl)-1-piperazinyl]propanesulfonic acid (HEPPSO), 2-(N-morpholino)ethanesulfonic acid (MES), 3-(N-morpholino)propanesulfonic acid (MOPS), 2-hydroxy-3-(N-morpholino)propanesulfonic acid (MOPSO), piperazine-1,4-bis(2-ethanesulfonic acid) (PIPES), piperazine-1,4-bis(2-hydroxy-3-propanesulfonic acid) (POPSO), N-tris(hydroxymethyl)methyl-3-aminopropanesulfonic acid (TAPS), 2-hydroxy-N-tris(hydroxymethyl)methyl-3-aminopropanesulfonic acid (TAPSO), N-tris(hydroxymethyl)methyl-2-aminoethanesulfonic acid (TES), and N-[tris(hydroxymethyl)methyl]glycine (Tricine), buffer solutions obtained by dissolving, for example, a phosphate, acetate, citrate, and tris(hydroxymethyl)aminomethane, a Veronal buffer, and a borate buffer solution. In addition, regarding these buffer solutions, one kind of buffer solution may be used alone, or two or more kinds of buffer solutions may be used in combination.

The biological specimen is not particularly limited, and examples thereof include blood-derived specimens such as serum, blood plasma, whole blood, and buffy coat, and body fluid specimens such as cerebrospinal fluid, urine, saliva, semen, thoracic exudate, tears, sputum, mucous, lymph, ascites, pleural fluid, amniotic fluid, bladder lavage fluid, and bronchovesicular lavage fluid, where blood-derived specimens are preferable, and serum or blood plasma is more preferable. In addition, it is preferable that the blood-derived specimens are those that have been subjected to lipid extraction (defatting treatment). As the biological specimen, for example, a collected biological specimen may be directly used or a biological specimen, which has been subjected to pretreatment such as recovery, concentration, purification, isolation, dilution with a buffer solution or the like, or filtration sterilization, may be used. These pretreatments may be appropriately carried out according to a conventional method.

### <Standard product for measuring hepcidin according to embodiment of present invention>

The standard product for measuring hepcidin according to the embodiment of the present invention contains hepcidin and a hepcidin adsorption inhibitor. The standard product for measuring hepcidin according to the embodiment of the present invention is used for quantifying hepcidin according to, for example, an analytical method such as an immunological measuring method or a mass spectrometry method. Specifically, the standard product for measuring hepcidin according to the embodiment of the present invention is used as a calibrator, a control, or the like, which is used for the creation of a calibration curve that shows a correlation between knowns amounts (concentrations) of hepcidin contained in the standard product and measured values obtained according to various analytical methods, the checking of the effectiveness of the accuracy control and calibration of analytical instruments, and the investigation of the stability of the temporal quantitative analysis. Examples of the measured value include an absorbance, a change in amount of absorbance, transmitted light, a change in amount of transmitted light, a fluorescence intensity, a change in amount of fluorescence intensity, a light emission amount, a change in amount of light emission amount, a turbidity, a rate of change in turbidity, scattered light, a rate of change in scattered light, a reflectivity, a change in amount of reflectivity, a refractive index, and a change in amount of refractive index.

The hepcidin and the hepcidin adsorption inhibitor in the standard product for measuring hepcidin according to the embodiment of the present invention are the same as those of the hepcidin adsorption inhibitor according to the embodiment of the present invention, and the same applies to specific examples and preferred ones thereof. The standard product for measuring hepcidin according to the embodiment of the present invention contains hepcidin and a hepcidin adsorption inhibitor, to which a value relating to the amount (concentration) of hepcidin is affixed, and it may be in a solution state or may be in a lyophilized state. The standard product for measuring hepcidin according to the embodiment of the present invention in a lyophilized state is obtained by lyophilizing a solution or suspension containing hepcidin and a hepcidin adsorption inhibitor, in a liquid such as water such as deionized water or distilled water, a buffer solution, a biological specimen, a liquid containing water or a buffer solution and a biological specimen, and in a case of being used, it is used in a solution state by water such as deionized water or distilled water, a buffer solution, a biological specimen, a liquid containing water or a buffer solution and a biological specimen, or the like. The standard product for measuring hepcidin according to the embodiment of the present invention in a solution state contains a known amount (concentration) of a hepcidin-containing liquid and a hepcidin adsorption inhibitor. The hepcidin-containing liquid is the same as that of the hepcidin adsorption inhibitor according to the embodiment of the present invention, and the same applies to specific examples and preferred ones thereof. The standard product for measuring hepcidin according to the embodiment of the present invention may contain other components in addition to the hepcidin and the hepcidin adsorption inhibitor. Examples of other components include a preservative (for example, sodium azide, salicylic acid, or benzoic acid), a reaction accelerator, an excipient, and a buffering agent.

The content of the hepcidin in the standard product for measuring hepcidin according to the embodiment of the present invention is, for example, 0.1 to 1,000 ng/mL, and it is preferably 1 to 500 ng/mL and more preferably 5 to 250 ng/mL. The content of the chelating agent in the standard product for measuring hepcidin according to the embodiment of the present invention is, for example, 0.01 to 500 mM, and it is preferably 0.1 to 50 mM and more preferably 1 to 10 mM.

### <Method of inhibiting adsorption of hepcidin according to embodiment of present invention>

The method of inhibiting adsorption of hepcidin according to the embodiment of the present invention is a method of inhibiting adsorption of hepcidin, which includes bringing a hepcidin-containing liquid into contact with at least one member selected from a rubber member, a plastic member, or a glass member, in a presence of a chelating agent.

In the method of inhibiting adsorption of hepcidin according to the embodiment of the present invention, at least one member (the member according to the embodiment of the present invention) selected from the chelating agent, the hepcidin-containing liquid, the rubber member, the plastic member, or the glass member is the same as that of the hepcidin adsorption inhibitor, and the same applies to preferred ones and specific examples thereof. In addition, the chelating agent in the method of inhibiting adsorption of hepcidin according to the embodiment of the present invention may be in a solid state or may be in a liquid state (hereinafter, a chelating agent-containing liquid according to the present invention), for example, in water such as deionized water or distilled water, a buffer solution, a biological specimen, or a solution or suspension contained in a liquid or the like, which contains water or a buffer solution, and a biological specimen. The buffer solution and the biological specimen are the same as those of the hepcidin adsorption inhibitor according to the embodiment of the present invention, and the same applies to preferred ones and specific examples thereof.

In the method of inhibiting adsorption of hepcidin according to the embodiment of the present invention, it suffices that "bringing the hepcidin-containing liquid into contact with the member according to the embodiment of the present invention in the presence of the chelating agent" is such that hepcidin comes into contact with the member according to the embodiment of the present invention in a liquid such as water such as deionized water or distilled water, a buffer solution, a biological specimen, a liquid containing water or a buffer solution and a biological specimen, in the presence of the chelating agent according to the embodiment of the present invention, where the contact order may be random. Specifically, the following method are mentioned; a method of bringing the chelating agent-containing liquid according to the present invention, the hepcidin-containing liquid, and the member according to the embodiment of the present invention into contact with each other, a method of bringing the chelating agent-containing liquid according to the present invention, the hepcidin, and the member according to the embodiment of the present invention into contact with each other, a method of bringing the chelating agent according to the embodiment of the present invention, the hepcidin-containing liquid, and the member according to the embodiment of the present invention into contact with each other, and a method of bringing the chelating agent according to the embodiment of the present invention, the hepcidin, and the member according to the embodiment of the present invention into contact with each other, in a liquid such as water such as deionized water or distilled water, a buffer solution, a biological specimen, a liquid containing water or a buffer solution and a biological specimen. The method of inhibiting adsorption of hepcidin according to the embodiment of the present invention may be carried out in the presence of, as other components, for example, a preservative (for example, sodium azide, salicylic acid, benzoic acid, or the like), a reaction accelerator, an excipient, and a buffering agent, in addition to the chelating agent, the hepcidin, and the member according to the embodiment of the present invention.

In the method of inhibiting adsorption of hepcidin according to the embodiment of the present invention, the content of hepcidin in "bringing the hepcidin-containing liquid into contact with the member according to the embodiment of the present invention in the presence of the chelating agent" is, for example, 0.1 to 1,000 ng/mL, and it is preferably 1 to 500 ng/mL and more preferably from 5 to 250 ng/mL. In the method of inhibiting adsorption of hepcidin according to the embodiment of the present invention, the amount (concentration) of the chelating agent in "bringing the hepcidin-containing liquid into contact with the member according to the embodiment of the present invention in the presence of the chelating agent" is not limited as long as it is an amount with which the adsorption of hepcidin is inhibited, and the amount thereof is, for example, 0.01 to 500 mM, and it is preferably 0.1 to 50 mM and more preferably 1 to 10 mM. In the method of inhibiting adsorption of hepcidin according to the embodiment of the present invention, the contact time in "bringing the hepcidin-containing liquid into contact with the member according to the embodiment of the present invention in the presence of the chelating agent" is, for example, from 1 second to 8 hours and preferably 1 minute to 1 hour, and the contact temperature is, for example, 0°C to 30°C and preferably 2°C to 25°C.

### <Reagent for measuring hepcidin according to embodiment of present invention>

The reagent for measuring hepcidin according to the embodiment of the present invention contains the hepcidin adsorption inhibitor according to the embodiment of the present invention. The reagent for measuring hepcidin according to the embodiment of the present invention is mainly used for measuring the amount of hepcidin in a biological specimen obtained from a test animal. Since the reagent for measuring hepcidin according to the embodiment of the present invention contains the hepcidin adsorption inhibitor according to the embodiment of the present invention, the adsorption of hepcidin is inhibited. Specifically, the same effect as that of the hepcidin adsorption inhibitor according to the embodiment of the present invention can be obtained.

In the reagent for measuring hepcidin according to the embodiment of the present invention, the content of the hepcidin adsorption inhibitor according to the embodiment of the present invention is not limited as long as it is an amount with which the adsorption of hepcidin is inhibited. The reagent for measuring hepcidin according to the embodiment of the present invention can contain, in addition to the hepcidin adsorption inhibitor according to the embodiment of the present invention, an optional component that is used for measuring hepcidin according to various measuring methods such as an immunological measuring method and a mass spectrometry method. Examples of the optional component include a diluted solution (specimen diluted solution) of a biological specimen derived from a test animal, an anti-hepcidin antibody, a carrier, a labeling substance, a buffer solution, an enzyme solution, or a substrate solution.

The anti-hepcidin antibody may be a commercially available product or an appropriately prepared antibody according to a conventional method, or it may be any one of a polyclonal antibody or a monoclonal antibody, where these may be used alone or in an appropriate combination thereof. In addition, not only the immunoglobulin molecule itself (intact immunoglobulin) but also a fragment thereof such as Fab, F(ab')2, or F(ab'), which is a fragment antibody having an ability to bind to an antigen, or a synthetic antibody such as a single chain antibody (single chain Fv), a diabody, a triabody, or a tetrabody may be used as the anti-hepcidin antibody. In addition, the preparation of these antibodies may be carried out, for example, according to the method described in "Immunoassay" (edited by Biochemical Assay Society of JAPAN, Kodansha, 2014).

### <Reagent kit for measuring hepcidin according to embodiment of present invention>

The reagent kit for measuring hepcidin according to the embodiment of the present invention includes a reagent kit in which the hepcidin adsorption inhibitor according to the embodiment of the present invention is accommodated in the measuring instrument according to the embodiment of the present invention. Since the reagent kit for measuring hepcidin according to the embodiment of the present invention uses the hepcidin adsorption inhibitor according to the embodiment of the present invention, the adsorption of hepcidin to the measuring instrument according to the present invention is inhibited, and thus more accurate measurement is possible.

Specific examples of the reagent kit for measuring hepcidin according to the embodiment of the present invention include a reagent kit in which the standard product for measuring hepcidin according to the embodiment of the present invention and/or the reagent for measuring hepcidin according to the embodiment of the present invention is accommodated in the measuring instrument according to the present invention. In the reagent kit for measuring hepcidin according to the embodiment of the present invention, the hepcidin adsorption inhibitor according to the embodiment of the present invention, the measuring instrument according to the present invention, and the standard product for measuring hepcidin according to the embodiment of the present invention are the same as those described above, and the same applies to preferred ones and specific examples thereof. In the reagent kit for measuring hepcidin according to the embodiment of the present invention, the hepcidin adsorption inhibitor according to the embodiment of the present invention which constitutes the reagent for measuring hepcidin according to the embodiment of the present invention and the optional component may be accommodated in the same measuring instrument according to the present invention or may be accommodated in independent containers, respectively.

In the present specification, "adsorption is inhibited" means that the adsorption of hepcidin to the member according to the embodiment of the present invention is inhibited in a case where the member according to the embodiment of the present invention and the hepcidin-containing liquid according to the present invention are brought into contact with each other. The inhibition of the adsorption can be checked by comparing the hepcidin concentrations in the hepcidin-containing liquid according to the present invention before and after the member according to the embodiment of the present invention and the hepcidin-containing liquid according to the present invention are brought into contact with each other. A measuring method for the hepcidin concentration is not particularly limited, and it is possible to measure the hepcidin concentration, for example, according to an immunological measuring method.

### <Measuring method for hepcidin according to embodiment of present invention>

In the measuring method for hepcidin according to the embodiment of the present invention, hepcidin is measured according to an analytical method such as an immunological measuring method or a mass spectrometry method by using the standard product for measuring hepcidin according to the embodiment of the present invention or/and the reagent for measuring hepcidin according to the embodiment of the present invention. In a case of using the standard product for measuring hepcidin according to the embodiment of the present invention or/and the reagent for measuring hepcidin according to the embodiment of the present invention, the adsorption of hepcidin is inhibited, and thus more accurate measurement is possible. The immunological measuring method may be any method as long as it is a method of carrying out an antigen-antibody reaction using an anti-hepcidin antibody. The measurement principle of the immunological measuring method is not particularly limited, and examples of the immunological measuring method include a sandwich method, a competition method, a coagulation method (immunonephelometry), immunochromatography, capillary electrophoresis, western blotting, and a surface plasmon resonance method (SPR method), where a coagulation method is preferable, and a latex agglutination method is more preferable. In addition, regarding the measurement principle, any one of a homogenous method or a heterogeneous method may be used. The labeling in the immunological measuring method is not particularly limited, and examples thereof include enzyme-linked immunosorbent assay (ELISA), enzyme immunoassay (EIA), radioimmunoassay (RIA), fluorescence enzyme immunoassay (FEIA), fluoroimmunoassay (FIA), chemiluminescence enzyme immunoassay (CLEIA), chemiluminescence immunoassay (CLIA), and electrochemical luminescence immunoassay (ECLIA).

### Examples

Hereinafter, the present invention will be specifically described based on Examples and Comparative Examples. However, the present invention is not limited to these examples.

### Experimental Example 1. Measurement of hepcidin 25 in hepcidin 25 solution containing no chelating agent

After preparing a hepcidin 25 solution containing no chelating agent, the concentration of hepcidin 25 in the solution was measured with the following reagent and according to the following method without dispensing (without transferring) the solution into a new container.

### (1) Preparation of hepcidin 25 solution (containing no chelating agent)

A hepcidin 25 solution containing no chelating agent [hereinafter, denoted as a hepcidin 25 solution (containing no chelating agent)], which had the following composition, was prepared in a centrifuge tube.
HEPES: 50 mmol/L (pH: 7.5), hepcidin 25 (manufactured by PEPTIDE INSTITUTE, INC.): 50 ng/mL, BSA (Probumin^{™} manufactured by Merck Millipore): 1 w/v%, water (solvent)

### (2) Hepcidin 25 quantification reagent

As a hepcidin 25 quantification reagent, the following first reagent (a buffer solution, R-1) and second reagent (a hepcidin 25 quantification reagent consisting of a latex particle-containing reagent sensitized with an anti-human hepcidin antibody, R-2) were prepared.
(i) First reagent (R-1)
   HEPES: 100 mmol/L (pH:7.85), sodium azide: 0.09%, BSA: 0.1 w/v%, Ethanaminium, N-(carboxymethyl)-N,N-dimethyl-2-[(2-methyl-1-oxo-2-propenyl)-oxy]-, inner salt, homopolymer (polymer for promoting coagulation: 22 w/v%, prepared according to the description in JP6107653B), water (solvent)
(ii) Second reagent (R-2)

As a first anti-human hepcidin antibody, a mouse monoclonal antibody was used, where the mouse monoclonal antibody was obtained by immunizing a mouse with an N-terminal hepcidin peptide (a peptide consisting of 7 amino acids from the N-terminal of hepcidin 25) to which Keyhole Limpet Hemocyanin (KLH) was bound and carrying out the murine iliac lymph node method described in JP4098796B. In addition, as a second anti-human hepcidin antibody, a rat monoclonal antibody was used, where the rat monoclonal antibody was obtained by immunizing a rat with hepcidin 25 to which BSA was bound and carrying out the iliac lymph node method. In a 50 mmol/L HEPES buffer solution (pH: 7.5), polystyrene latex particles (latex for reagent (manufactured by NIHON KOHDEN CORPORATION)) and the above-described two kinds of anti-human hepcidin antibodies were mixed, and the polystyrene latex particles were sensitized with the anti-human hepcidin antibody. Using the obtained anti-human hepcidin antibody-sensitized latex particles sensitized with the anti-human hepcidin antibody, a reagent containing the anti-human hepcidin antibody-sensitized latex particles, which had the following composition, was prepared.
HEPES: 50 mmol/L (pH: 7.1), BSA: 0.5 w/v%, anti-hepcidin antibody sensitized latex: 0.22 w/v%, water (solvent)

### (3) Creation of calibration curve

A solution for creating a calibration curve (a calibrator) having the following composition was prepared.
EDTA-2Na: 5 mmol/L, hepcidin 25 (manufactured by PEPTIDE INSTITUTE, INC., 12.7 ng/mL, 60.1 ng/mL, 110.5 ng/mL, 164.4 ng/mL, 232.9 ng/mL), serum (solvent)

Using the hepcidin 25 quantification reagents (the first reagent and the second reagent) prepared in (2), the absorbance of the solution for creating a calibration curve was measured with a TBA-c16000 automatic analyzer (manufactured by CANON MEDICAL SYSTEMS CORPORATION) under the following measurement conditions, thereby obtaining a calibration curve showing a relationship between the concentration and the absorbance of hepcidin 25. A physiological saline water was used for the measurement of the reagent blank.

### [Measurement conditions]

Analytical method/reaction time: [END UP]/[10]
Measurement points: [20], [21] to [30], [31]
Wavelength (secondary/main): [-] [804]
Specimen amount: 1.6 µL, R-1: 120 µL, R-2: 40 µL
Measurement temperature: 37°C

### (4) Measurement of absorbance

The absorbance of the hepcidin 25 solution (containing no chelating agent) prepared in (1) was measured with the same hepcidin 25 quantification reagent, device, and conditions as in the creation of the calibration curve of (3), and the hepcidin concentration in the hepcidin 25 solution (containing no chelating agent) was calculated using the calibration curve obtained in (3). The obtained result was used as a reference value (100%) for obtaining a relative value to the concentrations obtained in Comparative Examples 1 to 5.

### Comparative Example 1. Adsorption of hepcidin 25 to glass container

The adsorption of hepcidin 25 to a glass container (a vial bottle) was evaluated according to the following method. The concentration of hepcidin 25 in a specimen was measured, and a relative value was calculated according to the same method as in Experimental Example 1, except that, instead of "the hepcidin 25 solution (containing no chelating agent)", "a solution obtained by dispensing and stirring the hepcidin 25 solution (containing no chelating agent) prepared in the centrifuge tube in Experimental Example 1(1), in a glass container (a vial bottle) having a capacity of 12 mL" was used as a specimen. The obtained results are shown in Table 1 below. In the table, the column of "vs General measuring method" indicates a relative value of the concentration of hepcidin 25 which is obtained by each measurement, with respect to the concentration (100%) of hepcidin 25 obtained in Experimental Example 1.

### Comparative Example 2. Adsorption of hepcidin 25 to rubber lid

The adsorption of hepcidin 25 to a glass container (a vial bottle) having a rubber lid was evaluated according to the following method. The concentration of hepcidin 25 in a specimen was measured, and a relative value to the concentration of hepcidin 25 obtained in Experimental Example 1 was calculated according to the same method as in Experimental Example 1, except that, instead of "the hepcidin 25 solution (containing no chelating agent)", "a solution obtained by dispensing the hepcidin 25 solution (containing no chelating agent) prepared in the centrifuge tube in Experimental Example 1 (1), in a glass container (a vial bottle) having a capacity of 12 mL, and attaching a rubber lid (a butyl rubber stopper) thereto, followed by mixing with inversion by 30 times to bring the hepcidin 25 solution (containing no chelating agent) into contact with the rubber stopper" was used as a specimen. The obtained results are shown in Table 1 below.

### Comparative Examples 3 to 5. Adsorption of hepcidin 25 to plastic container

The adsorption of hepcidin 25 to a plastic container was evaluated according to the following method. The concentration of hepcidin 25 in a specimen was measured, and a relative value to the concentration of hepcidin 25 obtained in Experimental Example 1 was calculated according to the same method as in Experimental Example 1, except that, instead of "the hepcidin 25 solution (containing no chelating agent)", "each solution obtained by dispensing and stirring the hepcidin 25 solution (containing no chelating agent) prepared in the centrifuge tube in Experimental Example 1 (1), in a plastic container" was used as a specimen. As the plastic container, a "polystyrene container" was used in Comparative Example 3, a "polypropylene container" was used in Comparative Example 4, and a "polyethylene container" was used in Comparative Example 5. The obtained results are shown in Table 1 below.

**[Table 1]**

| | Measuring method | Chelating agent | vs General measuring method |
|---|---|---|---|
| Experimental Example 1 | General measuring method (measured without dispensing into a container) | Absent | 100% |
| Comparative Example 1 | Measured with dispensing into a glass container (vial bottle) and without mixing with inversion | Absent | 48% |
| Comparative Example 2 | Measured with dispensing into a glass container (vial bottle) after mixing with inversion in a state where a rubber stopper is attached | Absent | 17% |
| Comparative Example 3 | Measured after dispensing into a polystyrene container | Absent | 66% |
| Comparative Example 4 | Measured after dispensing into a polypropylene container | Absent | 78% |
| Comparative Example 5 | Measured after dispensing into a polyethylene container | Absent | 76% |

### Experimental Example 2. Measurement of hepcidin 25 in hepcidin 25 solution containing chelating agent

After preparing a hepcidin 25 solution containing a chelating agent, the concentration of hepcidin 25 in the solution was calculated without dispensing (without transferring) the solution into a new container.

The concentration of hepcidin 25 in a specimen was measured and calculated according to the same method as in Experimental Example 1, except that, instead of "the hepcidin 25 solution (containing no chelating agent)", "a hepcidin 25 solution (containing EDTA-2Na)" having the following composition was used as a specimen. The obtained result was used as a reference value (100%) for obtaining a relative value to the concentrations obtained in Examples 1 to 5.

Hepcidin 25 solution (containing EDTA-2Na):
HEPES: 50 mmol/L (pH: 7.5), hepcidin 25: 50 ng/mL, BSA: 1 w/v%, EDTA-2Na: 5 mmol/L, water (solvent)

### Example 1. Inhibition of adsorption of hepcidin 25 to glass container

The adsorption of hepcidin 25 to a glass container (a vial bottle) was evaluated according to the following method. The concentration of hepcidin 25 in a specimen was measured, and a relative value was calculated according to the same method as in Comparative Example 1, except that, instead of "the hepcidin 25 solution (containing no chelating agent)", "a hepcidin 25 solution (containing EDTA-2Na)" prepared according to the same method as in Experimental Example 2 was used as a specimen. The obtained results are shown in Table 2 below. In the table, the column of "vs General measuring method" indicates a relative value of the concentration of hepcidin 25 which is obtained by each measurement, with respect to the concentration (100%) of hepcidin 25 obtained in Experimental Example 2.

### Example 2. Inhibition of adsorption of adsorption of hepcidin 25 to rubber lid

The adsorption of hepcidin 25 to a glass container (a vial bottle) having a rubber lid was evaluated according to the following method. The concentration of hepcidin 25 in a specimen was measured, and a relative value was calculated according to the same method as in Comparative Example 2, except that, instead of "the hepcidin 25 solution (containing no chelating agent)", "a hepcidin 25 solution (containing EDTA-2Na)" prepared according to the same method as in Experimental Example 2 was used as a specimen. The obtained results are shown in Table 2 below.

### Examples 3 to 5. Inhibition of adsorption of hepcidin 25 to plastic container

The adsorption of hepcidin 25 to a plastic container was evaluated according to the following method. A concentration of hepcidin 25 in each specimen was measured, and a relative value was calculated according to the same method as in Comparative Examples 3 to 5, except that, instead of "the hepcidin 25 solution (containing no chelating agent)", "a hepcidin 25 solution (containing EDTA-2Na)" prepared according to the same method as in Experimental Example 2 was used as a specimen. As the plastic container, a "polystyrene container" was used in Example 3, a "polypropylene container" was used in Example 4, and a "polyethylene container" was used in Example 5. The obtained results are shown in Table 2 below.

**[Table 2]**

| | Measuring method | Chelating agent | vs General measuring method |
|---|---|---|---|
| Experimental Example 2 | General measuring method (measured without dispensing into a container) | EDTA-2Na | 100% |
| Example 1 | Measured with dispensing into a glass container (vial bottle) and without mixing with inversion | EDTA-2Na | 69% |
| Example 2 | Measured with dispensing into a glass container (vial bottle) after mixing with inversion in a state where a rubber stopper is attached | EDTA-2Na | 42% |
| Example 3 | Measured after dispensing into a polystyrene container | EDTA-2Na | 97% |
| Example 4 | Measured after dispensing into a polypropylene container | EDTA-2Na | 95% |
| Example 5 | Measured after dispensing into a polyethylene container | EDTA-2Na | 95% |

From Table 1, it was found that the hepcidin in the specimen containing no chelating agent is adsorbed to any member of the glass container, the rubber lid, or the plastic container. In addition, the adsorption of hepcidin to these members was insufficiently inhibited by BSA alone, which is a general adsorption inhibitor. In addition, from Table 2, it was found that the chelating agent (EDTA-2Na) inhibits the adsorption of hepcidin to the glass container, the rubber lid, and the plastic container.

### Experimental Example 3. Measurement of hepcidin 25 in hepcidin 25-containing serum containing no chelating agent

After preparing a hepcidin 25-containing serum containing no chelating agent, the concentration of the hepcidin 25 in the solution was calculated without dispensing (without transferring) the solution into a new container.

The concentration of hepcidin 25 in a specimen was measured and calculated according to the same method as in Experimental Example 1, except that, instead of "the hepcidin 25 solution (containing no chelating agent)", "a hepcidin 25-containing serum (containing no chelating agent)" having the following composition was used as a specimen. The obtained result was used as a reference value (100%) for obtaining a relative value to the concentrations obtained in Comparative Example 6.

Hepcidin 25-containing serum (containing no chelating agent):
defatted serum (solvent), hepcidin 25: 50 ng/mL

### Comparative Example 6. Inhibition of adsorption of adsorption of hepcidin 25 to rubber lid

The adsorption of hepcidin 25 to a glass container (a vial bottle) having a rubber lid was evaluated according to the following method. Specifically, The concentration of hepcidin 25 in a specimen was measured, and a relative value was calculated according to the same method as in Comparative Example 2, except that, instead of "the hepcidin 25 solution (containing no chelating agent)", "a hepcidin 25-containing serum (containing no chelating agent)" was used as a specimen. The obtained results are shown in Table 3 below. In the table, the column of "vs General measuring method" indicates a relative value of the concentration of hepcidin 25 which is obtained by the measurement, with respect to the concentration (100%) of hepcidin 25 obtained in Experimental Example 3.

**[Table 3]**

| | Measuring method | Chelating agent | vs General measuring method |
|---|---|---|---|
| Experimental Example 3 | General measuring method (measured without dispensing into a container) | Absent | 100% |
| Comparative Example 6 | Measured with dispensing into a glass container (vial bottle) after mixing with inversion in a state where a rubber stopper is attached | Absent | 83% |

### Experimental Example 4. Measurement of hepcidin 25 in hepcidin 25-containing serum containing chelating agent

After preparing a hepcidin 25-containing serum containing a chelating agent, the concentration of the hepcidin 25 in the solution was calculated without dispensing (without transferring) the solution into a new container.

Specifically, the concentration of hepcidin 25 in a specimen was measured and calculated according to the same method as in Experimental Example 3, except that, instead of "the hepcidin 25-containing serum (containing no chelating agent)", "a hepcidin 25 solution (containing EDTA-2Na)" was used as a specimen. The obtained result was used as a reference value (100%) for obtaining a relative value to the concentrations obtained in Example 6.

Hepcidin 25-containing serum (containing EDTA-2Na):
defatted serum (solvent), hepcidin 25: 50 ng/mL, EDTA-2Na: 5 mmol/L

### Example 6. Inhibition of adsorption of adsorption of hepcidin 25 to rubber lid

The adsorption of hepcidin 25 to a glass container (a vial bottle) having a rubber lid was evaluated according to the following method. Specifically, the concentration of hepcidin 25 in a specimen was measured and calculated according to the same method as in Comparative Example 2, except that, instead of "the hepcidin 25-containing serum (containing no chelating agent)", "a hepcidin 25 solution (containing EDTA-2Na)" after mixing with inversion was used as a specimen. The obtained results are shown in Table 4 below. In the table, the column of "vs General measuring method" indicates a relative value of the concentration of hepcidin 25 which is obtained by the measurement, with respect to the concentration (100%) of hepcidin 25 obtained in Experimental Example 4.

**[Table 4]**

| | Measuring method | Chelating agent | vs General measuring method |
|---|---|---|---|
| Experimental Example 4 | General measuring method (measured without dispensing into a container) | EDTA-2Na | 100% |
| Example 6 | Measured with dispensing into a glass container (vial bottle) after mixing with inversion in a state where a rubber stopper is attached | EDTA-2Na | 96% |

From Table 3 and Table 4, it was found that in the serum specimen containing no chelating agent, although hepcidin is adsorbed to the glass container including the rubber lid, the chelating agent (EDTA-2Na) inhibits the adsorption of hepcidin.

### Examples 7 to 14. Inhibition of adsorption of hepcidin 25 to plastic container

The adsorption of hepcidin 25 to a plastic container was evaluated according to the following method. Each "hepcidin 25 solution (containing a chelating agent)" was prepared according to the same method as in Experimental Example 2, except that the preparation was carried out to replace "5 mmol/L of EDTA-2Na" with "5 mmol/L of the chelating agent described in Table 5 below". In addition, the concentration of hepcidin 25 in each specimen was measured and calculated according to the same method as in Examples 3 to 5, except that the "hepcidin 25 solution (containing a chelating agent)" was used as a specimen, and the plastic container described in Table 5 below was used. The obtained results are shown in Table 5 below. The values in the table are relative values with respect to the concentration (100%) of hepcidin 25, which was measured without dispensing (without transferring) to a new container after preparing the hepcidin 25 solution (containing a chelating agent).

Hepcidin 25 solution (containing chelating agent):
HEPES: 50 mmol/L (pH: 7.5), hepcidin 25: 50 ng/mL, BSA: 1 w/v%, chelating agent: 5 mmol/L, water (solvent)

**[Table 5]**

| | | Example 7 | Example 8 | Example 9 | Example 10 | Example 11 | Example 12 | Example 13 | Example 14 |
|---|---|---|---|---|---|---|---|---|---|
| Chelating agent | | EDTPO | NTPO | NTA | BAPTA | CyDTA | GEDTA | DTPA | TTHA |
| Measuring method | Measured after dispensing into a polystyrene container | 94% | 99% | 94% | 96% | 98% | 94% | 96% | 93% |
| | Measured after dispensing into a polypropylene container | 93% | 96% | 92% | 96% | 95% | 92% | 94% | 93% |
| | Measured after dispensing into a polyethylene container | 91% | 95% | 93% | 90% | 92% | 92% | 92% | 95% |

From Table 5, it was found that the chelating agents EDTPO, NTPO, NTA, BAPTA, CyDTA, GEDTA, DTPA, and TTHA inhibit the adsorption of hepcidin to the plastic container.

### Examples 15 to 19. Inhibition of adsorption of adsorption of hepcidin 25 to rubber lid

The adsorption of hepcidin 25 to a glass container (a vial bottle) having a rubber lid was evaluated according to the following method. Each "hepcidin 25 serum (containing a chelating agent)" was prepared according to the same method as in Experimental Example 4 and used as a specimen, except that the preparation was carried out to replace "5 mmol/L of EDTA-2Na" with "5 mmol/L of the chelating agent described in Table 6 below". The concentration of hepcidin 25 in the specimen after mixing with inversion was measured and calculated according to the same method as in Example 6. The obtained results are shown in Table 6 below. The values in the table are relative values with respect to the concentration (100%) of hepcidin 25, which was measured without dispensing (without transferring) to a new container after preparing the hepcidin 25 serum (containing a chelating agent).

Hepcidin 25-containing serum (containing chelating agent):
defatted serum (solvent), hepcidin 25: 50 ng/mL, chelating agent: 5 mmol/L

**[Table 6]**

| | Example 15 | Example 16 | Example 17 | Example 18 | Example 19 |
|---|---|---|---|---|---|
| Chelating agent | EDTPO | NTPO | EDDA | NTA | BAPTA |
| Measured with dispensing into a glass container (vial bottle) after mixing with inversion in a state where a rubber stopper is attached | 94% | 95% | 97% | 94% | 96% |

From Table 6, it was found that according to the chelating agents EDTPO, NTPO, EDDA, NTA, and BAPTA, the adsorption of hepcidin to the glass container including the rubber lid is inhibited.

The hepcidin adsorption inhibitor, method of inhibiting adsorption, standard product, reagent, kit, and measuring method according to the embodiments of the present invention can inhibit the adsorption of hepcidin and thus are particularly useful in the field of clinical examination.

## Claims

1. A hepcidin adsorption inhibitor comprising:
a chelating agent.

2. The hepcidin adsorption inhibitor according to claim 1,
wherein the chelating agent is an aminocarboxylic acid-based chelating agent or a phosphonic acid-based chelating agent.

3. The hepcidin adsorption inhibitor according to claim 1 or 2,
wherein adsorption inhibition of the hepcidin adsorption inhibitor is inhibition of adsorption to a rubber member.

4. The hepcidin adsorption inhibitor according to claim 3,
wherein the rubber member is a synthetic rubber member.

5. The hepcidin adsorption inhibitor according to claim 4,
wherein the synthetic rubber member is a butyl rubber member.

6. The hepcidin adsorption inhibitor according to claim 1 or 2,
wherein adsorption inhibition of the hepcidin adsorption inhibitor is inhibition of adsorption to a plastic member or a glass member.

7. A method of inhibiting adsorption of hepcidin, comprising:
bringing a hepcidin-containing liquid into contact with at least one member selected from a rubber member, a plastic member, or a glass member, in a presence of a chelating agent.

8. The method of inhibiting adsorption of hepcidin according to claim 7,
wherein the chelating agent is an aminocarboxylic acid-based chelating agent or a phosphonic acid-based chelating agent.

9. The method of inhibiting adsorption of hepcidin according to claim 7 or 8,
wherein the member is a rubber member.

10. The method of inhibiting adsorption of hepcidin according to claim 9,
wherein the rubber member is a synthetic rubber member.

11. The method of inhibiting adsorption of hepcidin according to claim 10,
wherein the synthetic rubber member is a butyl rubber member.

12. The method of inhibiting adsorption of hepcidin according to claim 7 or 8,
wherein the member is a plastic member or a glass member.

13. A standard product for measuring hepcidin, comprising:
the hepcidin adsorption inhibitor according to any one of claims 1 to 6; and
hepcidin.

14. A reagent for measuring hepcidin, comprising:
the hepcidin adsorption inhibitor according to any one of claims 1 to 6.

15. A reagent kit for measuring hepcidin,
wherein the hepcidin adsorption inhibitor according to any one of claims 1 to 6 includes a hepcidin adsorption inhibitor that is accommodated in a measuring instrument having at least one member selected from a rubber member, a plastic member, or a glass member.

16. A measuring method for hepcidin,
wherein the standard product for measuring hepcidin according to claim 13 and/or the reagent for measuring hepcidin according to claim 14 is used.
